# EUROPÄISCHE PATENTSCHRIFT

(11) **EP 3 710 570 B1**
(45) Veröffentlichungstag und Bekanntmachung des Hinweises auf die Patenterteilung: **05.01.2022**
(21) Anmeldenummer: 18796647.8
(22) Anmeldetag: 02.11.2018
(51) Int. Cl.: C11D 3/37, C08F 16/14

(54) **WASCH- UND REINIGUNGSMITTEL MIT POLYMEREM WIRKSTOFF**
WASHING AND CLEANING AGENT WITH POLYMERIC ACTIVE INGREDIENT
AGENT DE LAVAGE ET DE NETTOYAGE À BASE D'UN PRINCIPE ACTIF POLYMÉRIQUE

(30) Priorität: 17.11.2017 DE 102017010653
(43) Veröffentlichungstag der Anmeldung: 23.09.2020
(73) Patentinhaber: Henkel AG & Co. KGaA, 40589 Düsseldorf (DE)
(72) Erfinder: LUNEAU, Benoit, 40885 Ratingen (DE); PALKOVITS, Regina, 52074 Aachen (DE); HAUSOUL, Peter, 6371 GE Landgraaf (NL); STOBBE, Carsten, 52068 Aachen (DE)
(86) Internationale Anmeldenummer: PCT/EP2018/079974
(87) Internationale Veröffentlichungsnummer: WO 2019/096592

(56) Entgegenhaltungen:
- WO-A1-2016/162253
- WO-A1-2016/162254
- JP-A- 2008 247 819
- ARCHANA R. PARAMESWAR ET AL: "Efficient stereoselective synthesis of oligosaccharides of Streptococcus pneumoniae serotypes 6A and 6B containing multiple 1,2-cis glycosidic linkages", TETRAHEDRON, Bd. 63, Nr. 40, 31. Dezember 2007 (2007-12-31), Seiten 10083-10091, XP055536559, AMSTERDAM, NL ISSN: 0040-4020, DOI: 10.1016/j.tet.2007.07.036

## Beschreibung

Die vorliegende Erfindung betrifft bestimmte aus nachwachsenden Rohstoffen zugängliche Polymere, die diesen zugrundeliegenden aus nachwachsenden Rohstoffen zugänglichen Monomere, die Verwendung derartiger Polymere zur Verstärkung der Primärwaschkraft von Wasch- oder Reinigungsmitteln beim Waschen von Textilien oder Reinigen harter Oberflächen, sowie Wasch- und Reinigungsmittel, welche derartige Polymere enthalten.

Waschmittel enthalten neben den für den Waschprozess unverzichtbaren Inhaltsstoffen wie Tensiden und Buildermaterialien in der Regel weitere Bestandteile, die man unter dem Begriff Waschhilfsstoffe zusammenfassen kann und die so unterschiedliche Wirkstoffgruppen wie Schaumregulatoren, Vergrauungsinhibitoren, Bleichmittel, Bleichaktivatoren und Farbübertragungsinhibitoren umfassen. Zu derartigen Hilfsstoffen gehören auch Substanzen, deren Anwesenheit die Waschkraft von Tensiden verstärkt, ohne dass sie in der Regel selbst ein ausgeprägtes tensidisches Verhalten aufzuweisen müssen. Gleiches gilt sinngemäß auch für Reinigungsmittel für harte Oberflächen. Derartige Substanzen werden oft als Waschkraftverstärker bezeichnet.

Aus der internationalen Patentanmeldung WO 01/57171 A1 sind Wasch- oder Nachspülmittel bekannt, die neben Tensid Copolymere aus anionischen und kationischen Monomeren sowie gegebenenfalls zusätzlich nichtionischen Monomeren enthalten. WO 2016/162254A1 offenbart das Einarbeiten von Polymeren mit sich von ethylenisch ungesättigten Carbonsäuren ableitenden Sulfobetaineinheiten um die Primärwaschkraft von Wasch- und Reinigungsmitteln sollte insbesondere gegenüber öl- und/oder fetthaltigen Anschmutzungen verbessert werden. WO 2016/162253A1 offenbart das Einarbeiten von Polymeren mit N-vinylcaprolactam und N-vinyl acetamide zur Lösung desselben Problems wie WO 2016/162254A1.

Derartige Copolymere weisen den Nachteil auf, dass sie im Wesentlichen vollständig aus Monomeren aufgebaut sind, die petrochemisch erzeugt werden. Es besteht ein Bedarf nach waschleistungsfördernden Polymeren, die zumindest anteilig aus Monomeren aufgebaut sind, welche aus nachwachsenden Rohstoffen hergestellt werden können.

Aus dem japanischen Patent JP 5288153 B sind Bisacetonide von 1,2,3,4,5-Pentolen bekannt, deren Hydroxylgruppe, die nicht in die Ketale überführt ist, eine Allyletherfunktion trägt.

Überraschenderweise wurde gefunden, dass Polymere von 3-Allyloxypentolen und Copolymere von 3-Allyloxypentolen mit ethylenisch ungesättigten Carbonsäuren besonders gute die Leistung von Wasch- und Reinigungsmitteln verstärkende Eigenschaften haben.

Ein erster Gegenstand der Erfindung ist 3-Allyloxy-1,2,4,5-tetrahydroxypentan und seine Derivate der allgemeinen Formel (I), 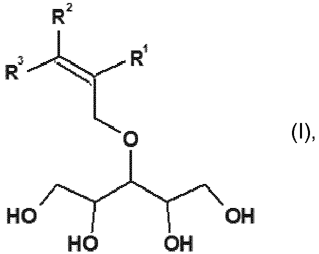 in denen R¹, R² und R³ unabhängig voneinander für H oder eine Alkylgruppe mit 1 bis 3 C-Atomen stehen, sowie deren Derivate, bei denen die Hydroxylgruppen durch Aufziehen üblicher Schutzgruppen, beispielsweise als Acetal wie Tetrahydropyranylether, als Ketal wie Acetonid oder als Carbonsäureester wie Acetat, geschützt sind.

Weitere Gegenstände der Erfindung sind Polymere, erhältlich durch radikalische Polymerisation von Verbindungen der vorgenannten allgemeinen Formel (I), und Copolymere, erhältlich durch radikalische Copolymerisation von Verbindungen der vorgenannten allgemeinen Formel (I), mit α,β-monoethylenisch ungesättigten Carbonsäuren, Carbonsäureestern, Carbonsäureanhydriden, Carbonsäureamiden, Carbonsäureimiden, Nitrilen und deren Mischungen. In bevorzugten Verbindungen der Formel I sind R¹, R² und R³ gleich; in ebenfalls bevorzugten Verbindungen der Formel I ist mindestens einer der Reste R¹, R² und R³ Wasserstoff.

Monomere der allgemeinen Formel I sind durch Umsetzung der mittleren Hydroxylgruppe von Xylitol oder Ribitol mit Allylierungsreagenzien, beispielsweise Allylbromid, zugänglich; dafür werden deren übrige Hydroxylgruppen zweckmäßigerweise in eine unreaktive Form überführt und beispielsweise säurekatalysiert als Acetonide geschützt, und aus diesen nach erfolgter Allylierung die Hydroxylgruppen in Position 1, 2, 4 und 5 wieder freigesetzt.

Sie können in Gegenwart üblicher Radikalstarter wie zum Beispiel Azobisisobutyronitril oder Benzoylperoxid polymerisiert oder mit ethylenisch ungesättigten Carbonsäuren oder Carbonsäurederivaten copolymerisiert werden, wobei die beiden vicinalen Hydroxylgruppenpaare bei der Polymerisation durch vorheriges Aufziehen üblicher Schutzgruppen, beispielsweise als Acetal wie Tetrahydropyranolether, als Ketal wie Acetonid oder als Carbonsäureester wie Acetat, geschützt werden können und man die Schutzgruppen nach der Polymerisation wieder entfernt.

Die α,ß-monoethylenisch ungesättigten Carbonsäuren und deren genannten Derivate werden vorzugsweise ausgewählt aus Säuren wie beispielsweise Acrylsäure, Methacrylsäure, Maleinsäure, Fumarsäure, Estern wie beispielsweise Dimethylmaleat, Dieethylmaleat, Dimethylfumarat, Diethylfumarat, Methylacrylat, Ethylacrylat, n-Propylacrylat, Isopropylacrylat, Butylacrylat, Pentylacrylat, Hexylacrylat, 2-Ethylhexylacrylat, Nonylacrylat, Laurylacrylat, Trimethylcyclohexylacrylat, t-Butylcyclohexylacrylat, Benzylacrylat, Hydroxyethylacrylat, Ethoxyethylacrylat, Ethoxyethoxyethylacrylat, Aminoethylacrylat, t-Butylaminoethylacrylat, N,N-Dimethylaminoethylacrylat, N,N-Diethyl-aminoethylacrylat, und entsprechende Methacrylate, Amiden wie beispielsweise Maleinsäurediamid, Fumarsäurediamid, Acrylamid, N-Methylacrylamid, N-Ethylacrylamid, N-n-Propylacrylamid, N-Isopropylacrylamid, N-Butylacrylamid, N-Octylacrylamid, N-Dodecylacrylamid, N-Octadecylacrylamid, N-Butoxymethylacrylamid, N,N-Dimethylacrylamid, N,N-Diethylacrylamid, N,N-Dipro-pylacrylamid, N,N-Dibutylacrylamid, N-(N',N'-Dimethylamino)ethylacrylamid, N-(N',N'-Diethyt-amino)ethylacrylamid, Methacrylamid und entsprechend N-substituierte Methacrylamide, Anhydriden wie beispielsweise Maleinsäureanhydrid, Imiden wie beispielsweise N-Acroyl- und N-Methacroylbutyro, -capro- und -valerolactam, Maleinimid, N-Phenyl- und N-Methyl-Maleinimid, Nitrilen wie beispielsweise Acetonitril und Fumarodinitril, die einzeln oder als Mischungen von zwei oder mehreren dieser Verbindungen eingesetzt werden können.

Ein weiterer Gegenstand der Erfindung ist die Verwendung von genannten Polymeren und/oder Copolymeren zur Verstärkung der Primärwaschkraft von Wasch- oder Reinigungsmitteln beim Waschen von Textilien oder beim Reinigen harter Oberflächen gegenüber Anschmutzungen. Insbesondere in diesen müssen die erfindungsgemäßen Copolymere nicht aus dem α,ß-monoethylenisch ungesättigten Monomer stammende Carbonsäuregruppen, Carbonsäureestergruppen, Carbonsäureanhydridgruppen, Carbonsäureamidgruppen oder Carbonsäureimidgruppen aufweisen, sondern diese können, ganz oder zumindest anteilig, hydrolysiert in Salzform, zum Beispiel als Natrium-, Kalium- oder Ammoniumcarboxylatgruppen, vorliegen, wobei die Ammoniumgruppe auch mit 1 bis 4 Alkyl- oder Hydroxyalkylgruppen oder deren Mischungen substituert sein kann.

Die erfindungswesentlichen Copolymere sind wie geschildert durch radikalische Copolymerisation der angegebenen Monomere, die als blockweise oder bevorzugt statistische Copolymerisation durchgeführt werden kann, zugänglich. Sie weisen außer aus den beiden genannten Monomeren stammenden Einheiten keine anderen Einheiten auf, wobei herstellungsbedingt an den Polymerenden aus dem Radikalstarter oder aus der Radikalabbruchreaktion stammende Einheiten anwesend sein können.

Der erfindungsgemäße polymere Wirkstoff weist vorzugsweise ein mittleres Molekulargewicht (hier und im Folgenden bei mittleren Molekulargewichtsangaben: Zahlenmittel) im Bereich von 1000 g/mol bis 100000 g/mol, insbesondere von 1500 g/mol bis 50000 g/mol auf. Im erfindungswesentlichen Copolymer liegen die aus der Verbindung der allgemeinen Formel I stammenden Einheiten und die aus der α,ß-monoethylenisch ungesättigten Carbonsäure und/oder deren Derivaten stammenden Einheiten vorzugsweise in Molverhältnissen im Bereich von 4:1 bis 1:4, insbesondere von 2:1 bis 1:2, vor.

Der Einsatz des erfindungsgemäß verwendeten Wirkstoffs führt zu einer signifikant besseren Ablösung von Anschmutzungen auf harten Oberflächen und auf Textilien, auch solchen aus Baumwolle oder mit einem Anteil von Baumwolle, als dies bei Verwendung bisher für diesen Zweck bekannter Verbindungen der Fall ist. Alternativ können bei gleich bleibendem Fettablösevermögen signifikante Mengen an Tensiden eingespart werden.

Die erfindungsgemäße Verwendung kann im Rahmen eines Wasch- oder Reinigungsprozesses derart erfolgen, dass man das erfindungswesentliche Polymer einer wasch- oder reinigungsmittelhaltigen wässrigen Flotte zusetzt oder es vorzugsweise als Bestandteil eines Wasch- oder Reinigungsmittels in die Flotte einbringt, wobei die Konzentration an dem Wirkstoff in der Flotte vorzugsweise im Bereich von 0,005 g/l bis 0,5 g/l, insbesondere von 0,02 g/l bis 0,1 g/l liegt.

Ein weiterer Gegenstand der Erfindung ist ein Verfahren zum Entfernen von Anschmutzungen von Textilien oder harten Oberflächen durch Kontaktieren des reinigungsbedürftigen Textils oder der reinigungsbedürftigen harten Oberfläche mit einer wässrigen Flotte, in der sich ein Wasch- oder Reinigungsmittel und ein genannter polymerer Wirkstoff befinden. Dieses Verfahren kann manuell oder maschinell, zum Beispiel mit Hilfe einer Haushaltswaschmaschine oder Geschirrspülmaschine, ausgeführt werden. Dabei ist es möglich, das insbesondere flüssige Wasch- oder Reinigungsmittel und den Wirkstoff gleichzeitig oder nacheinander anzuwenden. Die gleichzeitige Anwendung lässt sich besonders vorteilhaft durch den Einsatz eines Mittels, welches den Wirkstoff enthält, durchführen.

Ein weiterer Gegenstand der Erfindung ist daher ein Wasch- oder Reinigungsmittel, enthaltend ein oben definiertes erfindungsgemäßes Polymer.

Wasch- oder Reinigungsmittel, die einen erfindungsgemäß zu verwendenden Wirkstoff enthalten oder mit diesem zusammen verwendet oder im erfindungsgemäßen Verfahren eingesetzt werden, können alle üblichen sonstigen Bestandteile derartiger Mittel enthalten, die nicht in unerwünschter Weise mit dem erfindungswesentlichen Wirkstoff wechselwirken. Vorzugsweise wird ein oben definierter polymerer Wirkstoff in Mengen von 0,1 Gew.-% bis 10 Gew.-%, insbesondere 0,5 Gew.-% bis 2 Gew.-% in Wasch- oder Reinigungsmittel eingearbeitet.

Ein Mittel, welches einen erfindungsgemäß zu verwendenden Wirkstoff enthält oder mit diesem zusammen verwendet wird oder im erfindungsgemäßen Verfahren zum Einsatz kommt, enthält vorzugsweise synthetisches Aniontensid vom Sulfat- und/oder Sulfonattyp, insbesondere Alkylbenzolsulfonat, Fettalkylsulfat, Fettalkylethersulfat, Alkyl- und/oder Dialkylsulfosuccinat, Sulfofettsäureester und/oder Sulfofettsäuredisalze, insbesondere in einer Menge im Bereich von 2 Gew.-% bis 25 Gew.-% und besonders bevorzugt von 5 Gew.-% bis 15 Gew.-%. Bevorzugt wird das Aniontensid aus den Alkylbenzolsulfonaten, den Alkyl- oder Alkenylsulfaten und/oder den Alkyl- oder Alkenylethersulfaten ausgewählt, in denen die Alkyl- oder Alkenylgruppe 8 bis 22, insbesondere 12 bis 18 C-Atome besitzt. Bei diesen handelt es sich üblicherweise nicht um Einzelsubstanzen, sondern um Schnitte oder Mischungen. Darunter sind solche bevorzugt, deren Anteil an Verbindungen mit längerkettigen Resten im Bereich von 16 bis 18 C-Atomen über 20 Gew.-% beträgt. Besonders bevorzugt ist die Anwesenheit der oben genannten Kombination aus erfindungswesentlichem Polymer und Alkylbenzolsulfonat mit linearen C₉₋₁₃-Alkylgruppen in den Mitteln.

Eine weitere Ausführungsform derartiger Mittel umfasst die Anwesenheit von nichtionischem Tensid, ausgewählt aus Fettalkylpolyglykosiden, Fettalkylpolyalkoxylaten, insbesondere -ethoxylaten und/oder -propoxylaten, Fettsäurepolyhydroxyamiden und/oder Ethoxylierungs- und/oder Propoxylierungsprodukten von Fettalkylaminen, vicinalen Diolen, Fettsäurealkylestern und/oder Fettsäureamiden sowie deren Mischungen, insbesondere in einer Menge im Bereich von 2 Gew.-% bis 25 Gew.-%.

Zu den in Frage kommenden nichtionischen Tensiden gehören die Alkoxylate, insbesondere die Ethoxylate und/oder Propoxylate von gesättigten oder ein- bis mehrfach ungesättigten linearen oder verzweigtkettigen Alkoholen mit 10 bis 22 C-Atomen, vorzugsweise 12 bis 18 C-Atomen. Der Alkoxylierungsgrad der Alkohole liegt dabei in der Regel zwischen 1 und 20, vorzugsweise zwischen 3 und 10. Sie können in bekannter Weise durch Umsetzung der entsprechenden Alkohole mit den entsprechenden Alkylenoxiden hergestellt werden. Geeignet sind insbesondere die Derivate der Fettalkohole, obwohl auch deren verzweigtkettige Isomere, insbesondere sogenannte Oxoalkohole, zur Herstellung verwendbarer Alkoxylate eingesetzt werden können. Brauchbar sind demgemäß die Alkoxylate, insbesondere die Ethoxylate, primärer Alkohole mit linearen, insbesondere Dodecyl-, Tetradecyl-, Hexadecyl- oder Octadecyl-Resten sowie deren Gemische. Außerdem sind entsprechende Alkoxylierungsprodukte von Alkylaminen, vicinalen Diolen und Carbonsäureamiden, die hinsichtlich des Alkylteils den genannten Alkoholen entsprechen, verwendbar. Darüber hinaus kommen die Ethylenoxid- und/oder Propylenoxid-Insertionsprodukte von Fettsäurealkylestern sowie Fettsäurepolyhydroxyamide in Betracht. Zur Einarbeitung in die erfindungsgemäßen Mittel geeignete sogenannte Alkylpolyglykoside sind Verbindungen der allgemeinen Formel (G)ₙ-OR¹², in der R¹² einen Alkyl- oder Alkenylrest mit 8 bis 22 C-Atomen, G eine Glykoseeinheit und n eine Zahl zwischen 1 und 10 bedeuten. Bei der Glykosidkomponente (G)ₙ handelt es sich um Oligo- oder Polymere aus natürlich vorkommenden Aldose- oder Ketose-Monomeren, zu denen insbesondere Glucose, Mannose, Fruktose, Galaktose, Talose, Gulose, Altrose, Allose, Idose, Ribose, Arabinose, Xylose und Lyxose gehören. Die aus derartigen glykosidisch verknüpften Monomeren bestehenden Oligomere werden außer durch die Art der in ihnen enthaltenen Zucker durch deren Anzahl, den sogenannten Oligomerisierungsgrad, charakterisiert. Der Oligomerisierungsgrad n nimmt als analytisch zu ermittelnde Größe im allgemeinen gebrochene Zahlenwerte an; er liegt bei Werten zwischen 1 und 10, bei den vorzugsweise eingesetzten Glykosiden unter einem Wert von 1,5, insbesondere zwischen 1,2 und 1,4. Bevorzugter Monomer-Baustein ist wegen der guten Verfügbarkeit Glucose. Der Alkyl- oder Alkenylteil R¹² der Glykoside stammt bevorzugt ebenfalls aus leicht zugänglichen Derivaten nachwachsender Rohstoffe, insbesondere aus Fettalkoholen, obwohl auch deren verzweigtkettige Isomere, insbesondere sogenannte Oxoalkohole, zur Herstellung verwendbarer Glykoside eingesetzt werden können. Brauchbar sind demgemäß insbesondere die primären Alkohole mit linearen Octyl-, Decyl-, Dodecyl-, Tetradecyl-, Hexadecyl- oder Octadecylresten sowie deren Gemische. Besonders bevorzugte Alkylglykoside enthalten einen Kokosfettalkylrest, das heißt Mischungen mit im wesentlichen R¹²=Dodecyl und R¹²=Tetradecyl.

Nichtionisches Tensid ist in Mitteln, welche einen erfindungsgemäß verwendeten Wirkstoff enthalten oder im Rahmen der erfindungsgemäßen Verwendung eingesetzt werden, vorzugsweise in Mengen von 1 Gew.-% bis 30 Gew.-%, insbesondere von 1 Gew.-% bis 25 Gew.-% enthalten, wobei Mengen im oberen Teil dieses Bereiches eher in flüssigen Waschmitteln anzutreffen sind und teilchenförmige Waschmittel vorzugsweise eher geringere Mengen von bis zu 5 Gew.-% enthalten.

Die Mittel können stattdessen oder zusätzlich weitere Tenside, vorzugsweise synthetische Aniontenside des Sulfat- oder Sulfonat-Typs, enthalten. Als für den Einsatz in derartigen Mitteln besonders geeignete synthetische Aniontenside sind neben den bereits genannten Alkylbenzolsulfonaten die Alkyl- und/oder Alkenylsulfate mit 8 bis 22 C-Atomen, die ein Alkali-, Ammonium- oder Alkyl-oder Hydroxyalkyl-substituiertes Ammoniumion als Gegenkation tragen, zu nennen. Bevorzugt sind die Derivate der Fettalkohole mit insbesondere 12 bis 18 C-Atomen und deren verzweigtkettiger Analoga, der sogenannten Oxoalkohole. Die Alkyl- und Alkenylsulfate können in bekannter Weise durch Reaktion der entsprechenden Alkoholkomponente mit einem üblichen Sulfatierungsreagenz, insbesondere Schwefeltrioxid oder Chlorsulfonsäure, und anschließende Neutralisation mit Alkali-, Ammonium- oder Alkyl- oder Hydroxyalkyl-substituierten Ammoniumbasen hergestellt werden. Zu den einsetzbaren Tensiden vom Sulfat-Typ gehören auch die sulfatierten Alkoxylierungsprodukte der genannten Alkohole, sogenannte Ethersulfate. Vorzugsweise enthalten derartige Ethersulfate 2 bis 30, insbesondere 4 bis 10 Ethylenglykol-Gruppen pro Molekül. Zu den geeigneten Aniontensiden vom Sulfonat-Typ gehören die durch Umsetzung von Fettsäureestern mit Schwefeltrioxid und anschließender Neutralisation erhältlichen α-Sulfoester, insbesondere die sich von Fettsäuren mit 8 bis 22 C-Atomen, vorzugsweise 12 bis 18 C-Atomen, und linearen Alkoholen mit 1 bis 6 C-Atomen, vorzugsweise 1 bis 4 C-Atomen, ableitenden Sulfonierungsprodukte, sowie die durch formale Verseifung aus diesen hervorgehenden Sulfofettsäuren. Bevorzugte Aniontenside sind auch die Salze von Sulfobernsteinsäureestern, die auch als Alkylsulfosuccinate oder Dialkylsulfosuccinate bezeichnet werden, und die Monoester oder Diester der Sulfobernsteinsäure mit Alkoholen, vorzugsweise Fettalkoholen und insbesondere ethoxylierten Fettalkoholen darstellen. Bevorzugte Sulfosuccinate enthalten Cs- bis C₁₈-Fettalkoholreste oder Mischungen aus diesen. Insbesondere bevorzugte Sulfosuccinate enthalten einen ethoxylierten Fettalkoholrest, der für sich betrachtet ein nichtionisches Tenside darstellt. Dabei sind wiederum Sulfosuccinate, deren Fettalkohol-Reste sich von ethoxylierten Fettalkoholen mit eingeengter Homologenverteilung ableiten, besonders bevorzugt.

Als weitere fakultative tensidische Inhaltsstoffe kommen Seifen in Betracht, wobei gesättigte Fettsäureseifen, wie die Salze der Laurinsäure, Myristinsäure, Palmitinsäure oder Stearinsäure, sowie aus natürlichen Fettsäuregemischen, zum Beispiel Kokos-, Palmkern- oder Talgfettsäuren, abgeleitete Seifen geeignet sind. Insbesondere sind solche Seifengemische bevorzugt, die zu 50 Gew.-% bis 100 Gew.-% aus gesättigten C₁₂-C₁₈-Fettsäureseifen und zu bis 50 Gew.-% aus Ölsäureseife zusammengesetzt sind. Vorzugsweise ist Seife in Mengen von 0,1 Gew.-% bis 5 Gew.-% enthalten. Insbesondere in flüssigen Mitteln, welche einen erfindungsgemäß verwendeten Wirkstoff enthalten, können jedoch auch höhere Seifenmengen von in der Regel bis zu 20 Gew.-% enthalten sein.

Gewünschtenfalls können die Mittel auch Betaintenside und/oder kationische Tenside enthalten, die - falls vorhanden - vorzugsweise in Mengen von 0,5 Gew.-% bis 7 Gew.-% eingesetzt werden. Unter diesen sind die unten diskutierten Esterquats besonders bevorzugt.

Die Mittel können gewünschtenfalls Bleichmittel auf Persauerstoffbasis, insbesondere in Mengen im Bereich von 5 Gew.-% bis 70 Gew.-%, sowie gegebenenfalls Bleichaktivator, insbesondere in Mengen im Bereich von 2 Gew.-% bis 10 Gew.-%, enthalten. Die in Betracht kommenden Bleichmittel sind vorzugsweise die in Waschmitteln in der Regel verwendeten Persauerstoffverbindungen wie Percarbonsäuren, beispielsweise Dodecandipersäure oder Phthaloylaminoperoxicapronsäure, Wasserstoffperoxid, Alkaliperborat, das als Tetra- oder Monohydrat vorliegen kann, Percarbonat, Perpyrophosphat und Persilikat, die in der Regel als Alkalisalze, insbesondere als Natriumsalze, vorliegen. Derartige Bleichmittel sind in Waschmitteln, welche einen erfindungsgemäß verwendeten Wirkstoff enthalten, vorzugsweise in Mengen bis zu 25 Gew.-%, insbesondere bis zu 15 Gew.-% und besonders bevorzugt von 5 Gew.-% bis 15 Gew.-%, jeweils bezogen auf gesamtes Mittel, vorhanden, wobei insbesondere Percarbonat zum Einsatz kommt. Die fakultativ vorhandene Komponente der Bleichaktivatoren umfasst die üblicherweise verwendeten N- oder O-Acylverbindungen, beispielsweise mehrfach acylierte Alkylendiamine, insbesondere Tetraacetylethylendiamin, acylierte Glykolurile, insbesondere Tetraacetylglykoluril, N-acylierte Hydantoine, Hydrazide, Triazole, Urazole, Diketopiperazine, Sulfurylamide und Cyanurate, außerdem Carbonsäureanhydride, insbesondere Phthalsäureanhydrid, Carbonsäureester, insbesondere Natrium-isononanoyl-phenolsulfonat, und acylierte Zuckerderivate, insbesondere Pentaacetylglukose, sowie kationische Nitrilderivate wie Trimethylammoniumacetonitril-Salze. Die Bleichaktivatoren können zur Vermeidung der Wechselwirkung mit den Perverbindungen bei der Lagerung in bekannter Weise mit Hüllsubstanzen überzogen und/oder granuliert worden sein, wobei mit Hilfe von Carboxymethylcellulose granuliertes Tetraacetylethylendiamin mit mittleren Korngrößen von 0,01 mm bis 0,8 mm, granuliertes 1,5-Diacetyl-2,4-dioxohexahydro-1,3,5-triazin, und/oder in Teilchenform konfektioniertes Trialkylammoniumacetonitril besonders bevorzugt ist. In Waschmitteln sind derartige Bleichaktivatoren vorzugsweise in Mengen bis zu 8 Gew.-%, insbesondere von 2 Gew.-% bis 6 Gew.-%, jeweils bezogen auf gesamtes Mittel, enthalten.

In einer weiteren Ausführungsform enthält das Mittel wasserlöslichen und/oder wasserunlöslichen Builder, insbesondere ausgewählt aus Alkalialumosilikat, kristallinem Alkalisilikat mit Modul über 1, monomerem Polycarboxylat, polymerem Polycarboxylat und deren Mischungen, insbesondere in Mengen im Bereich von 2,5 Gew.-% bis 60 Gew.-%.

Das Mittel enthält vorzugsweise 20 Gew.-% bis 55 Gew.-% wasserlöslichen und/oder wasserunlöslichen, organischen und/oder anorganischen Builder. Zu den wasserlöslichen organischen Buildersubstanzen gehören insbesondere solche aus der Klasse der Polycarbonsäuren, insbesondere Citronensäure und Zuckersäuren, sowie der polymeren (Poly-)carbonsäuren, insbesondere die durch Oxidation von Polysacchariden zugänglichen Polycarboxylate, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative Molekülmasse der Homopolymeren ungesättigter Carbonsäuren liegt im allgemeinen zwischen 5000 g/mol und 200000 g/mol, die der Copolymeren zwischen 2000 g/mol und 200000 g/mol, vorzugsweise 50000 g/mol bis 120000 g/mol, bezogen auf freie Säure. Ein besonders bevorzugtes Acrylsäure-Maleinsäure-Copolymer weist eine relative Molekülmasse von 50000 g/mol bis 100000 g/mol auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei Carbonsäuren und/oder deren Salze sowie als drittes Monomer Vinylalkohol und/oder ein Vinylalkohol-Derivat oder ein Kohlenhydrat enthalten. Das erste saure Monomer oder dessen Salz leitet sich von einer monoethylenisch ungesättigten C₃-C₈-Carbonsäure und vorzugsweise von einer C₃-C₄-Monocarbonsäure, insbesondere von (Meth-)acrylsäure ab. Das zweite saure Monomer oder dessen Salz kann ein Derivat einer C₄-C₈-Dicarbonsäure sein, wobei Maleinsäure besonders bevorzugt ist. Die dritte monomere Einheit wird in diesem Fall von Vinylalkohol und/oder vorzugsweise einem veresterten Vinylalkohol gebildet. Insbesondere sind Vinylalkohol-Derivate bevorzugt, welche einen Ester aus kurzkettigen Carbonsäuren, beispielsweise von C₁-C₄-Carbonsäuren, mit Vinylalkohol darstellen. Bevorzugte Terpolymere enthalten dabei 60 Gew.-% bis 95 Gew.-%, insbesondere 70 Gew.-% bis 90 Gew.-% (Meth)acrylsäure und/oder (Meth)acrylat, besonders bevorzugt Acrylsäure und/oder Acrylat, und Maleinsäure und/oder Maleinat sowie 5 Gew.% bis 40 Gew.-%, vorzugsweise 10 Gew.-% bis 30 Gew.-% Vinylalkohol und/oder Vinylacetat. Ganz besonders bevorzugt sind dabei Terpolymere, in denen das Gewichtsverhältnis (Meth)acrylsäure und/oder (Meth)acrylat zu Maleinsäure und/oder Maleat zwischen 1:1 und 4:1, vorzugsweise zwischen 2:1 und 3:1 und insbesondere 2:1 und 2,5:1 liegt. Dabei sind sowohl die Mengen als auch die Gewichtsverhältnisse auf die Säuren bezogen. Das zweite saure Monomer oder dessen Salz kann auch ein Derivat einer Allylsulfonsäure sein, die in 2-Stellung mit einem Alkylrest, vorzugsweise mit einem C₁-C₄-Alkylrest, oder einem aromatischen Rest, der sich vorzugsweise von Benzol oder Benzol-Derivaten ableitet, substituiert ist. Bevorzugte Terpolymere enthalten dabei 40 Gew.-% bis 60 Gew.-%, insbesondere 45 bis 55 Gew.-% (Meth)acrylsäure und/oder (Meth)acrylat, besonders bevorzugt Acrylsäure und/oder Acrylat, 10 Gew.-% bis 30 Gew.-%, vorzugsweise 15 Gew.-% bis 25 Gew.-% Methallylsulfonsäure und/oder Methallylsulfonat und als drittes Monomer 15 Gew.-% bis 40 Gew.-%, vorzugsweise 20 Gew.-% bis 40 Gew.-% eines Kohlenhydrats. Dieses Kohlenhydrat kann dabei beispielsweise ein Mono-, Di-, Oligo- oder Polysaccharid sein, wobei Mono-, Di- oder Oligosaccharide bevorzugt sind, besonders bevorzugt ist Saccharose. Durch den Einsatz des dritten Monomers werden vermutlich Sollbruchstellen in dem Polymer eingebaut, die für die gute biologische Abbaubarkeit des Polymers verantwortlich sind. Diese Terpolymere weisen im Allgemeinen eine relative Molekülmasse zwischen 1000 g/mol und 200000 g/mol, vorzugsweise zwischen 2000 g/mol und 50000 g/mol und insbesondere zwischen 3000 g/mol und 10000 g/mol auf. Sie können, insbesondere zur Herstellung flüssiger Mittel, in Form wässriger Lösungen, vorzugsweise in Form 30- bis 50-gewichtsprozentiger wässriger Lösungen eingesetzt werden. Alle genannten Polycarbonsäuren werden in der Regel in Form ihrer wasserlöslichen Salze, insbesondere ihre Alkalisalze, eingesetzt.

Derartige organische Buildersubstanzen sind vorzugsweise in Mengen bis zu 40 Gew.-%, insbesondere bis zu 25 Gew.-% und besonders bevorzugt von 1 Gew.-% bis 5 Gew.-% enthalten. Mengen nahe der genannten Obergrenze werden vorzugsweise in pastenförmigen oder flüssigen, insbesondere wasserhaltigen, Mitteln eingesetzt.

Als wasserunlösliche, wasserdispergierbare anorganische Buildermaterialien werden insbesondere kristalline oder amorphe Alkalialumosilikate, in Mengen von bis zu 50 Gew.-%, vorzugsweise nicht über 40 Gew.-% und in flüssigen Mitteln insbesondere von 1 Gew.-% bis 5 Gew.-%, eingesetzt. Unter diesen sind die kristallinen Alumosilikate in Waschmittelqualität, insbesondere Zeolith NaA und gegebenenfalls NaX, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen liegt im Bereich von 100 bis 200 mg CaO pro Gramm. Geeignete Substitute oder Teilsubstitute für das genannte Alumosilikat sind kristalline Alkalisilikate, die allein oder im Gemisch mit amorphen Silikaten vorliegen können. Die in den Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Derartige amorphe Alkalisilikate sind beispielsweise unter dem Namen Portil^{®} im Handel erhältlich.

Solche mit einem molaren Verhältnis Na₂O:SiO₂ von 1:1,9 bis 1:2,8 werden im Rahmen der Herstellung bevorzugt als Feststoff und nicht in Form einer Lösung zugegeben. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ · yH₂O eingesetzt, in der x, das sogenannte Modul, eine Zahl von 1,9 bis 4 und y eine Zahl von 0 bis 20 ist und bevorzugte Werte für x 2, 3 oder 4 sind. Kristalline Schichtsilikate, die unter diese allgemeine Formel fallen, werden beispielsweise in der europäischen Patentanmeldung EP 0 164 514 beschrieben. Bevorzugte kristalline Schichtsilikate sind solche, bei denen x in der genannten allgemeinen Formel die Werte 2 oder 3 annimmt. Insbesondere sind sowohl ß- als auch δ-Natriumdisitikate (Na₂Si₂O₅·yH₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in Mitteln, welche einen erfindungsgemäß zu verwendenden Wirkstoff enthalten, eingesetzt werden. In einer weiteren bevorzugten Ausführungsform erfindungsgemäßer Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5, werden in einer weiteren bevorzugten Ausführungsform von Waschmitteln, welche einen erfindungsgemäß verwendeten Wirkstoff enthalten, eingesetzt. Deren Gehalt an Alkalisilikaten beträgt vorzugsweise 1 Gew.% bis 50 Gew.-% und insbesondere 5 Gew.-% bis 35 Gew.-%, bezogen auf wasserfreie Aktivsubstanz. Falls als zusätzliche Buildersubstanz auch Alkalialumosilikat, insbesondere Zeolith, vorhanden ist, beträgt der Gehalt an Alkalisilikat vorzugsweise 1 Gew.-% bis 15 Gew.-% und insbesondere 2 Gew.-% bis 8 Gew.-%, bezogen auf wasserfreie Aktivsubstanz. Das Gewichtsverhältnis Alumosilikat zu Silikat, jeweils bezogen auf wasserfreie Aktivsubstanzen, beträgt dann vorzugsweise 4:1 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Zusätzlich zum genannten anorganischen Builder können weitere wasserlösliche oder wasserunlösliche anorganische Substanzen in den Mitteln, welche einen erfindungsgemäß zu verwendenden Wirkstoff enthalten, mit diesem zusammen verwendet oder in erfindungsgemäßen Verfahren eingesetzt werden, enthalten sein. Geeignet sind in diesem Zusammenhang die Alkalicarbonate, Alkalihydrogencarbonate und Alkalisulfate sowie deren Gemische. Derartiges zusätzliches anorganisches Material kann in Mengen bis zu 70 Gew.-% vorhanden sein.

Zusätzlich können die Mittel weitere in Wasch- oder Reinigungsmitteln übliche Bestandteile enthalten. Zu diesen fakultativen Bestandteilen gehören insbesondere Enzyme, Enzymstabilisatoren, Komplexbildner für Schwermetalle, beispielsweise Aminopolycarbonsäuren, Aminohydroxypolycarbonsäuren, Polyphosphonsäuren und/oder Aminopolyphosphonsäuren, Schauminhibitoren, beispielsweise Organopolysiloxane oder Paraffine, Lösungsmittel und optische Aufheller, beispielsweise Stilbendisulfonsäurederivate. Vorzugsweise sind in Mitteln, welche einen erfindungsgemäß verwendeten Wirkstoff enthalten, bis zu 1 Gew.-%, insbesondere 0,01 Gew.-% bis 0,5 Gew.-% optische Aufheller, insbesondere Verbindungen aus der Klasse der substituierten 4,4'-Bis-(2,4,6-tri-amino-s-triazinyl)-stilben-2,2'-disulfonsäuren, bis zu 5 Gew.-%, insbesondere 0,1 Gew.-% bis 2 Gew.-% Komplexbildner für Schwermetalle, insbesondere Aminoalkylenphosphonsäuren und deren Salze und bis zu 2 Gew.-%, insbesondere 0,1 Gew.-% bis 1 Gew.-% Schauminhibitoren enthalten, wobei sich die genannten Gewichtsanteile jeweils auf gesamtes Mittel beziehen.

Lösungsmittel, die insbesondere bei flüssigen Mitteln eingesetzt werden können, sind neben Wasser vorzugsweise solche, die wassermischbar sind. Zu diesen gehören die niederen Alkohole, beispielsweise Ethanol, Propanol, iso-Propanol, und die isomeren Butanole, Glycerin, niedere Glykole, beispielsweise Ethylen- und Propylenglykol, und die aus den genannten Verbindungsklassen ableitbaren Ether. In derartigen flüssigen Mitteln liegen die erfindungsgemäß verwendeten Wirkstoffe in der Regel gelöst oder in suspendierter Form vor.

Gegebenenfalls anwesende Enzyme werden vorzugsweise aus der Gruppe umfassend Protease, Amylase, Lipase, Cellulase, Hemicellulase, Oxidase, Peroxidase, Pektinase und Mischungen aus diesen ausgewählt. In erster Linie kommt aus Mikroorganismen, wie Bakterien oder Pilzen, gewonnene Protease in Frage. Sie kann in bekannter Weise durch Fermentationsprozesse aus geeigneten Mikroorganismen gewonnen werden. Proteasen sind im Handel beispielsweise unter den Namen BLAP^{®}, Savinase^{®}, Esperase^{®}, Maxatase^{®}, Optimase^{®}, Alcalase^{®}, Durazym^{®} oder Maxapem^{®} erhältlich. Die einsetzbare Lipase kann beispielsweise aus Humicola lanuginosa, aus Bacillus-Arten, aus Pseudomonas-Arten, aus Fusarium-Arten, aus Rhizopus-Arten oder aus Aspergillus-Arten gewonnen werden. Geeignete Lipasen sind beispielsweise unter den Namen Lipo-Iase^{®}, Lipozym^{®}, Lipomax^{®}, Lipex^{®}, Amano^{®}-Lipase, Toyo-Jozo^{®}-Lipase, Meito^{®}-Lipase und Diosynth^{®}-Lipase im Handel erhältlich. Geeignete Amylasen sind beispielsweise unter den Namen Maxamyl^{®}, Termamyl^{®}, Duramyl^{®} und Purafect^{®} OxAm handelsüblich. Die einsetzbare Cellulase kann ein aus Bakterien oder Pilzen gewinnbares Enzym sein, welches ein pH-Optimum vorzugsweise im schwach sauren bis schwach alkalischen Bereich von 6 bis 9,5 aufweist. Derartige Cellulasen sind unter den Namen Celluzyme^{®}, Carezyme^{®} und Ecostone^{®} handelsüblich. Geeignete Pektinasen sind beispielsweise unter den Namen Gamanase^{®}, Pektinex AR^{®}, X-Pect^{®} oder Pectaway^{®} von Novozymes, unter dem Namen Rohapect UF^{®}, Rohapect TPL^{®}, Rohapect PTE100^{®}, Rohapect MPE^{®}, Rohapect MA plus HC, Rohapect DA12L^{®}, Rohapect 10L^{®}, Rohapect B1L^{®} von AB Enzymes und unter dem Namen Pyrolase^{®} von Diversa Corp., San Diego, CA, USA erhältlich.

Zu den gegebenenfalls, insbesondere in flüssigen Mitteln vorhandenen üblichen Enzymstabilisatoren gehören Aminoalkohole, beispielsweise Mono-, Di-, Triethanol- und -propanolamin und deren Mischungen, niedere Carbonsäuren, Borsäure, Alkaliborate, Borsäure-Carbonsäure-Kombinationen, Borsäureester, Boronsäurederivate, Calciumsalze, beispielsweise Ca-Ameisensäure-Kombination, Magnesiumsalze, und/oder schwefelhaltige Reduktionsmittel.

Zu den geeigneten Schauminhibitoren gehören langkettige Seifen, insbesondere Behenseife, Fettsäureamide, Paraffine, Wachse, Mikrokristallinwachse, Organopolysiloxane und deren Gemische, die darüber hinaus mikrofeine, gegebenenfalls silanierte oder anderweitig hydrophobierte Kieselsäure enthalten können. Zum Einsatz in partikelförmigen Mitteln sind derartige Schauminhibitoren vorzugsweise an granulare, wasserlösliche Trägersubstanzen gebunden.

Zu den bekanntlich polyesteraktiven schmutzablösevermögenden Polymeren, die zusätzlich zu den erfindungswesentlichen Wirkstoffen eingesetzt werden können, gehören Copolyester aus Dicarbonsäuren, beispielsweise Adipinsäure, Phthalsäure oder Terephthalsäure, Diolen, beispielsweise Ethylenglykol oder Propylenglykol, und Polydiolen, beispielsweise Polyethylenglykol oder Polypropylenglykol. Zu den bevorzugt eingesetzten schmutzablösevermögenden Polyestern gehören solche Verbindungen, die formal durch Veresterung zweier Monomerteile zugänglich sind, wobei das erste Monomer eine Dicarbonsäure HOOC-Ph-COOH und das zweite Monomer ein Diol HO-(CHR¹¹-)ₐOH, das auch als polymeres Diol H-(O-(CHR¹¹-)ₐ)_{b}OH vorliegen kann, ist. Darin bedeutet Ph einen o-, m- oder p-Phenylenrest, der 1 bis 4 Substituenten, ausgewählt aus Alkylresten mit 1 bis 22 C-Atomen, Sulfonsäuregruppen, Carboxylgruppen und deren Mischungen, tragen kann, R¹¹ Wasserstoff, einen Alkylrest mit 1 bis 22 C-Atomen und deren Mischungen, a eine Zahl von 2 bis 6 und b eine Zahl von 1 bis 300. Vorzugsweise liegen in den aus diesen erhältlichen Polyestern sowohl Monomerdioleinheiten -O-(CHR¹¹-)ₐO- als auch Polymerdioleinheiten -(O-(CHR¹¹-)ₐ)_{b}O- vor. Das molare Verhältnis von Monomerdioleinheiten zu Polymerdioleinheiten beträgt vorzugsweise 100:1 bis 1:100, insbesondere 10:1 bis 1:10. In den Polymerdioleinheiten liegt der Polymerisationsgrad b vorzugsweise im Bereich von 4 bis 200, insbesondere von 12 bis 140. Das Molekulargewicht oder das mittlere Molekulargewicht oder das Maximum der Molekulargewichtsverteilung bevorzugter schmutzablösevermögender Polyester liegt im Bereich von 250 g/mol bis 100000 g/mol, insbesondere von 500 g/mol bis 50000 g/mol. Die dem Rest Ph zugrundeliegende Säure wird vorzugsweise aus Terephthalsäure, Isophthalsäure, Phthalsäure, Trimellithsäure, Mellithsäure, den Isomeren der Sulfophthalsäure, Sulfoisophthalsäure und Sulfoterephthalsäure sowie deren Gemischen ausgewählt. Sofern deren Säuregruppen nicht Teil der Esterbindungen im Polymer sind, liegen sie vorzugsweise in Salzform, insbesondere als Alkali- oder Ammoniumsalz vor. Unter diesen sind die Natrium- und Kaliumsalze besonders bevorzugt. Gewünschtenfalls können statt des Monomers HOOC-Ph-COOH geringe Anteile, insbesondere nicht mehr als 10 Mol-% bezogen auf den Anteil an Ph mit der oben gegebenen Bedeutung, anderer Säuren, die mindestens zwei Carboxylgruppen aufweisen, im schmutzablösevermögenden Polyester enthalten sein. Zu diesen gehören beispielsweise Alkylen- und Alkenylendicarbonsäuren wie Malonsäure, Bernsteinsäure, Fumarsäure, Maleinsäure, Glutarsäure, Adipinsäure, Pimelinsäure, Korksäure, Azelainsäure und Sebacinsäure. Zu den bevorzugten Diolen HO-(CHR¹¹-)ₐOH gehören solche, in denen R¹¹ Wasserstoff und a eine Zahl von 2 bis 6 ist, und solche, in denen a den Wert 2 aufweist und R¹¹ unter Wasserstoff und den Alkylresten mit 1 bis 10, insbesondere 1 bis 3 C-Atomen ausgewählt wird. Unter den letztgenannten Diolen sind solche der Formel HO-CH₂-CHR¹¹-OH, in der R¹¹ die obengenannte Bedeutung besitzt, besonders bevorzugt. Beispiele für Diolkomponenten sind Ethylenglykol, 1,2-Propylenglykol, 1,3-Propylenglykol, 1,4-Butandiol, 1,5-Pentandiol, 1,6-Hexandiol, 1,8-Octandiol, 1,2-Decandiol, 1,2-Dodecandiol und Neopentylglykol. Besonders bevorzugt unter den polymeren Diolen ist Polyethylenglykol mit einer mittleren Molmasse im Bereich von 1000 g/mol bis 6000 g/mol.

Gewünschtenfalls können diese wie oben beschrieben zusammengesetzten Polyester auch endgruppenverschlossen sein, wobei als Endgruppen Alkylgruppen mit 1 bis 22 C-Atomen und Ester von Monocarbonsäuren in Frage kommen. Den über Esterbindungen gebundenen Endgruppen können Alkyl-, Alkenyl- und Arylmonocarbonsäuren mit 5 bis 32 C-Atomen, insbesondere 5 bis 18 C-Atomen, zugrundeliegen. Zu diesen gehören Valeriansäure, Capronsäure, Önanthsäure, Caprylsäure, Pelargonsäure, Caprinsäure, Undecansäure, Undecensäure, Laurinsäure, Lauroleinsäure, Tridecansäure, Myristinsäure, Myristoleinsäure, Pentadecansäure, Palmitinsäure, Stearinsäure, Petroselinsäure, Petroselaidinsäure, Ölsäure, Linolsäure, Linolaidinsäure, Linolensäure, Eläostearinsäure, Arachinsäure, Gadoleinsäure, Arachidonsäure, Behensäure, Erucasäure, Brassidinsäure, Clupanodonsäure, Lignocerinsäure, Cerotinsäure, Melissinsäure, Benzoesäure, die 1 bis 5 Substituenten mit insgesamt bis zu 25 C-Atomen, insbesondere 1 bis 12 C-Atomen tragen kann, beispielsweise tert.-Butylbenzoesäure. Den Endgruppen können auch Hydroxymonocarbonsäuren mit 5 bis 22 C-Atomen zugrundeliegen, zu denen beispielsweise Hydroxyvaleriansäure, Hydroxycapronsäure, Ricinolsäure, deren Hydrierungsprodukt Hydroxystearinsäure sowie o-, m- und p-Hydroxybenzoesäure gehören. Die Hydroxymonocarbonsäuren können ihrerseits über ihre Hydroxylgruppe und ihre Carboxylgruppe miteinander verbunden sein und damit mehrfach in einer Endgruppe vorliegen. Vorzugsweise liegt die Anzahl der Hydroxymonocarbonsäureeinheiten pro Endgruppe, das heißt ihr Oligomerisierungsgrad, im Bereich von 1 bis 50, insbesondere von 1 bis 10. In einer bevorzugten Ausgestaltung der Erfindung werden Polymere aus Ethylenterephthalat und Polyethylenoxid-terephthalat, in denen die Polyethylenglykol-Einheiten Molgewichte von 750 g/mol bis 5000 g/mol aufweisen und das Molverhältnis von Ethylenterephthalat zu Polyethylenoxid-terephthalat 50:50 bis 90:10 beträgt, in Kombination mit einem erfindungswesentlichen Wirkstoff verwendet.

Die schmutzablösevermögenden Polymere sind vorzugsweise wasserlöslich, wobei unter dem Begriff "wasserlöslich" eine Löslichkeit von mindestens 0,01 g, vorzugsweise mindestens 0,1 g des Polymers pro Liter Wasser bei Raumtemperatur und pH 8 verstanden werden soll. Bevorzugt eingesetzte Polymere weisen unter diesen Bedingungen jedoch eine Löslichkeit von mindestens 1 g pro Liter, insbesondere mindestens 10 g pro Liter auf.

Die Herstellung erfindungsgemäßer fester Mittel bietet keine Schwierigkeiten und kann auf bekannte Weise, zum Beispiel durch Sprühtrocknen oder Granulation, erfolgen, wobei Enzyme und eventuelle weitere thermisch empfindliche Inhaltsstoffe wie zum Beispiel Bleichmittel gegebenenfalls später separat zugesetzt werden. Zur Herstellung erfindungsgemäßer Mittel mit erhöhtem Schüttgewicht, insbesondere im Bereich von 650 g/l bis 950 g/l, ist ein einen Extrusionsschritt aufweisendes Verfahren bevorzugt.

Zur Herstellung von erfindungsgemäßen Mitteln in Tablettenform, die einphasig oder mehrphasig, einfarbig oder mehrfarbig und insbesondere aus einer Schicht oder aus mehreren, insbesondere aus zwei Schichten bestehen können, geht man vorzugsweise derart vor, dass man alle Bestandteile - gegebenenfalls je einer Schicht - in einem Mischer miteinander vermischt und das Gemisch mittels herkömmlicher Tablettenpressen, beispielsweise Exzenterpressen oder Rundläuferpressen, mit Preßkräften im Bereich von etwa 50 bis 100 kN, vorzugsweise bei 60 bis 70 kN verpreßt. Insbesondere bei mehrschichtigen Tabletten kann es von Vorteil sein, wenn mindestens eine Schicht vorverpreßt wird. Dies wird vorzugsweise bei Preßkräften zwischen 5 und 20 kN, insbesondere bei 10 bis 15 kN durchgeführt. Man erhält so problemlos bruchfeste und dennoch unter Anwendungsbedingungen ausreichend schnell lösliche Tabletten mit Bruch- und Biegefestigkeiten von normalerweise 100 bis 200 N, bevorzugt jedoch über 150 N. Vorzugsweise weist eine derart hergestellte Tablette ein Gewicht von 10 g bis 50 g, insbesondere von 15 g bis 40 g auf. Die Raumform der Tabletten ist beliebig und kann rund, oval oder eckig sein, wobei auch Zwischenformen möglich sind. Ecken und Kanten sind vorteilhafterweise abgerundet. Runde Tabletten weisen vorzugsweise einen Durchmesser von 30 mm bis 40 mm auf. Insbesondere die Größe von eckig oder quaderförmig gestalteten Tabletten, welche überwiegend über die Dosiervorrichtung beispielsweise der Geschirrspülmaschine eingebracht werden, ist abhängig von der Geometrie und dem Volumen dieser Dosiervorrichtung. Beispielhaft bevorzugte Ausführungsformen weisen eine Grundfläche von (20 bis 30 mm) × (34 bis 40 mm), insbesondere von 26x36 mm oder von 24x38 mm auf.

Flüssige oder pastöse erfindungsgemäße Mittel in Form von übliche Lösungsmittel, insbesondere Wasser, enthaltenden Lösungen werden in der Regel durch einfaches Mischen der Inhaltsstoffe, die in Substanz oder als Lösung in einen automatischen Mischer gegeben werden können, hergestellt.

In einer bevorzugten Ausführungsform ist ein Mittel, in das erfindungsgemäß zu verwendender Wirkstoff eingearbeitet wird, flüssig und enthält 1 Gew.-% bis 15 Gew.-%, insbesondere 2 Gew.-% bis 10 Gew.-% nichtionisches Tensid, 2 Gew.-% bis 30 Gew.-%, insbesondere 5 Gew.-% bis 20 Gew.-% synthetisches Aniontensid, bis zu 15 Gew.-%, insbesondere 2 Gew.-% bis 12,5 Gew.-% Seife, 0,5 Gew.-% bis 5 Gew.-%, insbesondere 1 Gew.-% bis 4 Gew.-% organischen Builder, insbesondere Polycarboxylat wie Citrat, bis zu 1,5 Gew.-%, insbesondere 0,1 Gew.-% bis 1 Gew.-% Komplexbildner für Schwermetalle, wie Phosphonat, und neben gegebenenfalls enthaltenem Enzym, Enzymstabilisator, Farb- und/oder Duftstoff Wasser und/oder wassermischbares Lösungsmittel.

In einer weiteren bevorzugten Ausführungsform ist ein Mittel, in das erfindungsgemäß zu verwendender Wirkstoff eingearbeitet wird, teilchenförmig und enthält bis zu 25 Gew.-%, insbesondere 5 Gew.-% bis 20 Gew.-% Bleichmittel, insbesondere Alkalipercarbonat, bis zu 15 Gew.-%, insbesondere 1 Gew.-% bis 10 Gew.-% Bleichaktivator, 20 Gew.-% bis 55 Gew.-% anorganischen Builder, bis zu 10 Gew.-%, insbesondere 2 Gew.-% bis 8 Gew.-% wasserlöslichen organischen Builder, 10 Gew.-% bis 25 Gew.-% synthetisches Aniontensid, 1 Gew.-% bis 5 Gew.-% nichtionisches Tensid und bis zu 25 Gew.-%, insbesondere 0,1 Gew.-% bis 25 Gew.-% anorganische Salze, insbesondere Alkalicarbonat und/oder -hydrogencarbonat.

### Beispiele

### Beispiel 1: Monomerherstellung

### a) Herstellung von (4-(2,2-dimethyl-1,3-dioxolan-4-yl)hydroxymethyl)-2,2-dimethyl-1,3-dioxolan

Eine Mischung von 50,00 g Xylitol (328,6 mmol) mit 350 ml Aceton (4,76 mol, 5 15 Äq) und 100 µl 98-gewichtsprozentiger wässriger H₂SO₄ wurde für 72 h auf 90 °C erhitzt. Dann wurden 2 Spatel NaHCO₃ zugesetzt und der Überschuss an diesem Neutralisationsmitel abfiltriert. Das neutralisierte Reaktionsgemisch wurde am Rotationsverdampfer eingeengt, bis ein leicht gelber Sirup zurück blieb. Dieser wurde bei 150 °C im Hochvakuum (2,0×10⁻³ mbar) destilliert. 59,8 g Produkt wurden als farbloser Sirup mit einer Ausbeute von 79 % erhalten.
δ [¹H, CDCl₃] = 1,30 (d, 3H); 1,36 (d, 6H); 3,49-3,62 (m, 1H); 3,68-3,85 (m, 2H); 3,85-4,03 (m, 2H); 4,11 (dtd, 1H) ppm
δ [¹³C, CDCl₃] = 25,16; 25,40; 26,09; 26,32; 26,90; 27,06; 62,11; 65,53; 65,86; 71,53; 75,05; 75,97; 76,89; 77,72; 109,42; 109,53; 109,66 ppm

### b) Herstellung von 4-(Allyloxy-(2,2-dimethyl-1,3-dioxolan-4-yl)methyl)-2,2-dimethyl-1,3-dioxolan

Eine Lösung von 15,1 g (64,9 mmol) des in Beispiel 1a) erhaltenen Bisacetonids in 40 ml Dimethylformamid (DMF) wurde portionsweise mit 1,40 g Natriumhydrid (58,4 mmol, 0,9 Äq.) versetzt und für ca. 5 min gerührt, bis keine Bläschenbildung mehr erkennbar war. Dann wurde das Reaktionsgemisch auf 0 °C gekühlt, tropfenweise mit 7,3 ml Allylbromid (84,4 mmol, 1,3 Äq.) versetzt und 48 h bei Raumtemperatur gerührt. Anschließend wurde das Reaktionsgemisch mit 30 ml vollentsalztem Wasser und 2 × 30 ml Diethylether extrahiert. Die vereinigten organischen Phasen wurden mit jeweils 20 ml gesättigter NaHCO₃ und gesättigter NaCl gewaschen und am Rotationsverdampfer bis zu einem gelben Sirup eingeengt. Dieser wure im Hochvakuum (1,8 × 10⁻³ mbar) bei 100 °C destilliert. Man erhielt 15,0 g 4-(Allyloxy-(2,2-dimethyl-1,3-dioxolan-4-yl)methyl)-2,2-dimethyl-1,3-dioxolan als farblosen Sirup, Ausbeute 85 %.
δ [¹H, CDCl₃] = 1,19-1,35 (m, 12H); 3,38-3,50 (m, 2H); 3,66-3,82 (m, 2H); 3,84-4,01 (m, 4H); 4,01-4,14 (m, 1H); 5,06 (dt, 1H); 5,15 (dq, 1H); 5,76 (ddt, 1H) ppm
δ [¹³C, CDCl₃] = 25,26; 25,43; 26,15; 26,21; 26,87; 26,97; 65,60; 65,91; 70,54; 72,40; 75,53; 76,33; 78,38; 108,91; 109,47; 109,57; 116,93; 117,14; 134,28 ppm

### Beispiel 2: Polymerherstellung

### a) Homopolymerisation

Eine Lösung von 740 mg des in Beispiel 1b) hergestellten Bisacetonids (2,7 mmol) und 9,6 mg Benzoylperoxid (BPO, 0,04 mmol, 1,3 Gew.-% bezogen auf das Monomer) in 2 ml Toluol wurde unter Durchleiten von Stickstoff entgast und anschließend im verschlossenen Kolben unter N₂ für 23 h auf 70°C erhitzt. Weitere 23 mg BPO (0,09 mmol) wurden zugegeben und die Reaktion wurde bei 80°C für ca. 96 h fortgesetzt. Anschließend wurden erneut 67,2 mg BPO (0,27 mmol) zugegeben und die Reaktion wurde bei 80°C für weitere ca. 96 h fortgesetzt. Danach wurde das entstandene Polymer durch Eintropfen in Methanol / Wasser (30:70 / v:v) ausgefällt.

60,5 mg des Polymers wurden in 10 ml Dichlormethan gelöst, mit 2 ml Trifluoressigsäure und einigen Tropfen H₂O versetzt und zur Entfernung der Schutzgruppen für 2 Tage bei Raumtemperatur gerührt. Das Polymer wurde durch Gelpermeationschromatographie (Eluent DMF mit 0,1% LiBr) charakterisiert. Zur Molmassenkalibrierung wurden Polystyrolstandards verwendet.

Mₙ = 2300 g·mol⁻¹, M_{w} = 7000 g·mol⁻¹.

### b) Copolymerisation mit Maleinsäureanhydrid

Eine Lösung von 308 mg des in Beispiel 1b) hergestellten Bisacetonids (1,1 mmol), 115 mg Maleinsäureanhydrid (1,2 mmol) und 12 mg Azobisisobutyronitril (AIBN, 0,07 mmol, 3,0 Gew.-% bezogen auf das Allyloxy-Monomer) in 1 ml Methanol wurde unter Durchleiten von Stickstoff entgast und anschließend im verschlossenen Kolben unter N₂ für ca. 18 h auf 65°C erhitzt. Dann wurden weitere 36,2 mg AIBN (0,22 mmol, 8,6 Gew.-%) zugegeben und die Reaktion wurde bei 65°C für ca. 117 h fortgesetzt. Der Umsatz liegt nach diesem Schritt etwa bei 75%. Durch Eintropfen der Reaktionsmischung in 25 ml Diethylether wurde das Polymer ausgefällt.

60,5 mg des Polymers wurden in 10 ml Dichlormethan gelöst, mit 2 ml Trifluoressigsäure und einigen Tropfen H₂O versetzt und zur Entfernung der Schutzgruppen für 2 Tage bei Raumtemperatur gerührt. Das Polymer wurde durch Gelpermeationschromatographie (Eluent DMF mit 0,1% LiBr) charakterisiert. Zur Molmassenkalibrierung wurden Polystyrolstandards verwendet.

Mₙ = 7000 g·mol⁻¹, M_{w} = 15000 g·mol⁻¹.

### c) Copolymerisation mit Methylmaleimid

Eine Lösung von 308 mg des in Beispiel 1b) hergestellten Bisacetonids (1,1 mmol), 126 mg Methylmaleimid (1,1 mmol) und 12 mg Azobis-isobutyronitril (AIBN, 0,07 mmol, 3,0 Gew.-% bezogen auf Allyloxy-Monomer) in 1 ml Toluol wurde unter Durchleiten von Stickstoff entgast und anschließend im verschlossenen Kolben unter N₂ für ca. 18 h auf 80°C erhitzt. Dann wurden weitere 36 mg AIBN (0,22 mmol, 8,3 Gew.-%) zugegeben und die Reaktion wurde bei 80°C für ca. 23 h fortgesetzt. Danach wurde das Polymer durch Eintropfen in 20 ml Diethylether gefällt. Das gefällte Polymer wurde durch GPC in Dimethylformamid (DMF) mit 0,1% LiBr charakterisiert. Zur Molmassenkalibrierung wurden Polystyrolstandards verwendet.

60,5 mg des Polymers wurden in 10 ml Dichlormethan gelöst, mit 2 ml Trifluoressigsäure und einigen Tropfen H₂O versetzt und zur Entfernung der Schutzgruppen für 2 Tage bei Raumtemperatur gerührt. Das Polymer wurde durch Gelpermeationschromatographie (Eluent DMF mit 0,1% LiBr) charakterisiert. Zur Molmassenkalibrierung wurden Polystyrolstandards verwendet.

Mₙ = 7000 g·mol⁻¹, M_{w} = 12000 g·mol⁻¹.

### Beispiel 3: Waschversuche

Waschmittel mit erfindungsgemäß zu verwendenden Wirkstoffen zeigten eine deutlich bessere Primärwaschleistung als ansonsten gleich zusammengesetzte Mittel, denen diese fehlten, oder ansonsten gleich zusammengesetzte Mittel, die stattdessen ein Polymer des Standes der Technik enthielten.

## Patentansprüche

1. Polymer, erhältlich durch radikalische Polymerisation von Verbindungen der allgemeinen Formel I, 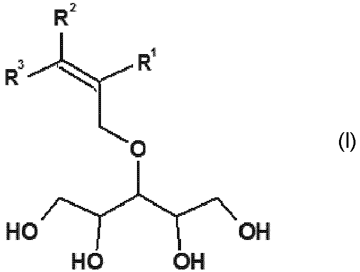 in der R¹, R² und R³ unabhängig voneinander für H oder eine Alkylgruppe mit 1 bis 3 C-Atomen stehen, oder durch deren radikalische Copolymersiation mit α,ß-monoethylenisch ungesättigten Carbonsäuren, Carbonsäureestern, Carbonsäureanhydriden, Carbonsäureamiden, Carbonsäureimiden, Nitrilen und deren Mischungen.

2. Verwendung von Polymeren oder Copolymeren gemäß Anspruch 1 zur Verstärkung der Primärwaschkraft von Wasch- oder Reinigungsmitteln beim Waschen von Textilien oder beim Reinigen harter Oberflächen gegenüber Anschmutzungen.

3. Verwendung nach Anspruch 2, **dadurch gekennzeichnet, dass** man das Polymer und/oder das Copolymer einer wasch- oder reinigungsmittelhaltigen wässrigen Flotte zusetzt oder es als Bestandteil eines Wasch- oder Reinigungsmittels in die Flotte einbringt, wobei die Konzentration an Polymer und/oder Copolymer in der Flotte im Bereich von 0,005 g/l bis 0,5 g/l, insbesondere von 0,02 g/l bis 0,1 g/l liegt.

4. Verfahren zum Entfernen von Anschmutzungen von Textilien oder harten Oberflächen durch Kontaktieren des reinigungsbedürftigen Textils oder der reinigungsbedürftigen harten Oberfläche mit einer wässrigen Flotte, in der sich ein Wasch- oder Reinigungsmittel und ein Polymer oder Copolymer gemäß Anspruch 1 befindet.

5. Wasch- oder Reinigungsmittel, enthaltend ein Polymer oder Copolymer gemäß Anspruch 1.

6. Mittel nach Anspruch 5, **dadurch gekennzeichnet, dass** es 0,1 Gew.-% bis 10 Gew.-%, insbesondere 0,5 Gew.-% bis 2 Gew.-% des Polymers und/oder Copolymers enthält.

7. Polymer, Copolymer, Verwendung, Verfahren oder Mittel nach einem der vorangehenden Ansprüche, **dadurch gekennzeichnet, dass** das Polymer und/oder Copolymer ein zahlenmittleres Molekulargewicht im Bereich von 1000 g/mol bis 100000 g/mol, insbesondere von 1500 g/mol bis 50000 g/mol aufweist, wobei das zahlenmittlere Molekulargewicht nach der in der Spezifikation genannten Methode bestimmt wird.

8. Copolymer, Verwendung, Verfahren oder Mittel nach einem der vorangehenden Ansprüche, **dadurch gekennzeichnet, dass** in dem Copolymer die aus der Verbindung der allgemeinen Formel I stammenden Einheiten und die aus der α,ß-monoethylenisch ungesättigten Carbonsäure und/oder deren Derivaten stammenden Einheiten vorzugsweise in Molverhältnissen im Bereich von 4:1 bis 1:4, insbesondere von 2:1 bis 1:2 vorliegen.

9. Copolymer, Verwendung, Verfahren oder Mittel nach einem der vorangehenden Ansprüche, **dadurch gekennzeichnet, dass** die α,ß-monoethylenisch ungesättigten Carbonsäuren und deren Derivate aus Säuren wie Acrylsäure, Methacrylsäure, Maleinsäure, Fumarsäure, Estern wie beispielsweise Dimethylmaleat, Dieethylmaleat, Dimethylfumarat, Dieethylfumarat, Methylacrylat, Ethylacrylat, n-Propylacrylat, Isopropylacrylat, Butylacrylat, Pentylacrylat, Hexylacrylat, 2-Ethylhexylacrylat, Nonylacrylat, Laurylacrylat, Trimethylcyclohexylacrylat, t-Butylcyclohexylacrylat, Benzylacrylat, Hydroxyethylacrylat, Ethoxyethylacrylat, Ethoxyethoxyethylacrylat, Aminoethylacrylat, t-Butylaminoethylacrylat, N,N-Dimethylaminoethylacrylat, N,N-Diethylaminoethylacrylat, und entsprechende Methacrylate, Amiden wie Maleinsäurediamid, Fumarsäurediamid, Acrylamid, N-Methylacrylamid, N-Ethylacrylamid, N-n-Propylacrylamid, N-Isopropylacrylamid, N-Butylacrylamid, N-Octylacrylamid, N-Dodecylacrylamid, N-Octadecylacrylamid, N-Butoxymethylacrylamid, N,N-Dimethylacrylamid, N,N-Di-ethylacrylamid, N,N-Dipropylacrylamid, N,N-Dibutylacrylamid, N-(N',N'-Dimethylami-no)ethylacrylamid, N-(N',N'-Diethylamino)ethylacrylamid, Methacrylamid und entsprechend N-substituierte Methacrylamide, Anhydriden wie Maleinsäureanhydrid, Imiden wie N-Acroyl-und N-Methacroylbutyro, -capro- und -valerolactam, Maleinimid, N-Phenyl- und N-Methyl-Maleinimid, Nitrilen wie beispielsweise Acetonitril und Fumarodinitril, die einzeln oder als Mischungen von zwei oder mehreren dieser Verbindungen vorliegen können, ausgewählt werden, und/oder dass in der Verbindung der Formel I R², R³ und R⁴ gleich sind und/oder in der Verbindung der Formel I mindestens einer der Reste R², R³ und R⁴ Wasserstoff ist, und/oder dass die aus dem α,ß-monoethylenisch ungesättigten Monomer stammenden Carbonsäuregruppen, Carbonsäureestergruppen, Carbonsäureanhydridgruppen, Carbonsäureamidgruppen oder Carbonsäureimidgruppen, ganz oder zumindest anteilig, hydrolysiert in Salzform vorliegen.

## Claims

1. A polymer, obtainable by radical polymerization of compounds of general formula I, 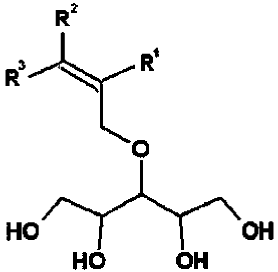 in which R¹, R², and R³ represent, independently of one another, H or an alkyl group having 1 to 3 C atoms, or by the radical copolymerization of said compounds with α,β-monoethylenically unsaturated carboxylic acids, carboxylic acid esters, carboxylic acid anhydrides, carboxylic acid amides, carboxylic acid imides, nitriles, and mixtures thereof.

2. The use of polymers or copolymers according to claim 1 for increasing the primary detergency of washing or cleaning agents when washing textiles or when cleaning hard surfaces of dirt.

3. The use according to claim 2, **characterized in that** the polymer and/or the copolymer is added to an aqueous liquor containing a washing or cleaning agent or is introduced into the liquor as a component of a washing or cleaning agent, the concentration of polymer and/or copolymer in the liquor being in the range of 0.005 g/l to 0.5 g/l, in particular 0.02 g/l to 0.1 g/l.

4. A method for removing dirt from textiles or hard surfaces by bringing the textile in need of cleaning or the hard surface in need of cleaning into contact with an aqueous liquor which contains a washing or cleaning agent and a polymer or copolymer according to claim 1.

5. A washing or cleaning agent containing a polymer or copolymer according to claim 1.

6. The agent according to claim 5, **characterized in that** it contains 0.1 wt.% to 10 wt.%, in particular 0.5 wt.% to 2 wt.% of the polymer and/or copolymer.

7. The polymer, copolymer, use, method, or agent according to one of the preceding claims, **characterized in that** the polymer and/or copolymer has a number average molecular weight in the range of 1000 g/mol to 100,000 g/mol, in particular 1500 g/mol to 50,000 g/mol, the number average molecular weight being determined according to the method mentioned in the specification.

8. The copolymer, use, method, or agent according to one of the preceding claims, **characterized in that** the units originating from the compound of general formula I and the units originating from the α,β-monoethylenically unsaturated carboxylic acid and/or derivatives thereof are preferably present in the copolymer in molar ratios in the range of 4:1 to 1:4, in particular 2:1 to 1:2.

9. The copolymer, use, method or agent according to one of the preceding claims, **characterized in that** the α,β-monoethylenically unsaturated carboxylic acids and derivatives thereof are selected from acids such as acrylic acid, methacrylic acid, maleic acid, fumaric acid, esters such as, for example, dimethyl maleate, diethyl maleate, dimethyl fumarate, diethyl fumarate, methyl acrylate, ethyl acrylate, n-propyl acrylate, isopropyl acrylate, butyl acrylate, pentyl acrylate, hexyl acrylate, 2-ethylhexyl acrylate, nonyl acrylate, lauryl acrylate, trimethylcyclohexyl acrylate, t-butylcyclohexyl acrylate, benzyl acrylate, hydroxyethyl acrylate, ethoxyethyl acrylate, ethoxy ethoxyethyl acrylate, aminoethyl acrylate, t-butylaminoethyl acrylate, N,N-dimethylaminoethyl acrylate, N,N-diethylaminoethyl acrylate, and corresponding methacrylates, amides such as maleic acid diamide, fumaric acid diamide, acrylamide, N-methylacrylamide, N-ethylacrylamide, N-n-propylacrylamide, N-isopropylacrylamide, N-butylacrylamide, N-octylacrylamide, N-dodecylacrylamide, N-octadecylacrylamide, N-butoxymethylacrylamide, N,N-dimethylacrylamide, N,N-di-ethylacrylamide, N,N-dipropylacrylamide, N,N-dibutylacrylamide, N-(N',N'-dimethylamino)ethylacrylamide, N-(N',N'-diethylamino)ethylacrylamide, methacrylamide, and corresponding N-substituted methacrylamides, anhydrides such as maleic anhydride, imides such as N-acroyl and N-methacroyl butyro-, capro- and valerolactam, maleimide, N-phenyl and N-methyl maleimide, and nitriles such as acetonitrile and fumaronitrile, which can be present individually or as mixtures of two or more of said compounds, and/or **in that** R², R³, and R⁴ in the compound of formula I are identical and/or at least one of the functional groups R², R³, and R⁴ in the compound of formula I is hydrogen, and/or **in that** the carboxylic acid groups, carboxylic acid ester groups, carboxylic acid anhydride groups, carboxylic acid amide groups, or carboxylic acid imide groups originating from the α,β-monoethylenically unsaturated monomer are present entirely or at least partially hydrolyzed in salt form.

## Revendications

1. Polymère pouvant être obtenu par polymérisation par voie radicalaire de composés de formule générale I, 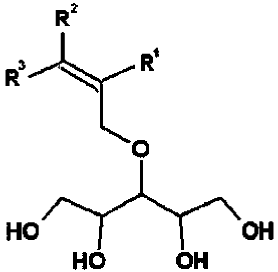 dans laquelle R¹, R² et R³ représentent indépendamment les uns des autres H ou un groupe alkyle comportant 1 à 3 atomes de carbone, ou, par leur copolymérisation radicalaire, comportant des acides carboxyliques à insaturation α,β-monoéthylénique, des esters d'acides carboxyliques, des anhydrides d'acides carboxyliques, des amides d'acides carboxyliques, des imides d'acides carboxyliques, des nitriles et leurs mélanges.

2. Utilisation de polymères ou copolymères selon la revendication 1 pour augmenter la puissance de lavage primaire d'agents de lavage ou d'agents de nettoyage contre les salissures lors du lavage de textiles ou lors du nettoyage de surfaces dures.

3. Utilisation selon la revendication 2, **caractérisée en ce que** le polymère et/ou le copolymère sont ajoutés à un bain aqueux contenant un agent de lavage ou un agent de nettoyage ou sont introduits dans le bain en tant que composant d'un agent de lavage ou d'un agent de nettoyage, la concentration en polymère et/ou en copolymère dans le bain se trouvant dans la plage de 0,005 g/l à 0,5 g/l, en particulier de 0,02 g/l à 0,1 g/l.

4. Procédé permettant l'élimination de salissures de textiles ou de surfaces dures par mise en contact du textile à nettoyer ou de la surface dure à nettoyer au moyen d'un bain aqueux dans lequel se trouvent un agent de lavage ou un agent de nettoyage et un polymère ou un copolymère selon la revendication 1.

5. Agent de lavage ou agent de nettoyage contenant un polymère ou un copolymère selon la revendication 1.

6. Agent selon la revendication 5, **caractérisé en ce qu'**il contient le polymère et/ou le copolymère en une quantité de 0,1 % en poids à 10 % en poids, en particulier de 0,5 % en poids à 2 % en poids.

7. Polymère, copolymère, utilisation, procédé ou agent selon l'une des revendications précédentes, **caractérisé en ce que** le polymère et/ou le copolymère présentent un poids moléculaire moyen en nombre dans la plage de 1 000 g/mol à 100 000 g/mol, en particulier de 1 500 g/mol à 50 000 g/mol, le poids moléculaire moyen en nombre étant déterminé selon la méthode mentionnée dans la spécification.

8. Copolymère, utilisation, procédé ou agent selon l'une des revendications précédentes, **caractérisé en ce que** dans le copolymère, les motifs provenant du composé de formule générale I et les motifs provenant de l'acide carboxylique à insaturation α,ß-monoéthylénique et/ou de ses dérivés se trouvent de préférence dans des rapports molaires dans la plage de 4:1 à 1:4, en particulier de 2:1 à 1:2.

9. Copolymère, utilisation, procédé ou agent selon l'une des revendications précédentes, **caractérisé en ce que** les acides carboxyliques à insaturation α,β-monoéthylénique et leurs dérivés sont choisis parmi les acides tels que l'acide acrylique, l'acide méthacrylique, l'acide maléique, l'acide fumarique, les esters tels que par exemple le maléate de diméthyle, le maléate de diéthyle, le fumarate de diméthyle, le fumarate de diéthyle, l'acrylate de méthyle, l'acrylate d'éthyle, l'acrylate de n-propyle, l'acrylate d'isopropyle, l'acrylate de butyle, l'acrylate de pentyle, l'acrylate d'hexyle, l'acrylate de 2-éthylhexyle, le nonylacrylate, l'acrylate de lauryle, l'acrylate de triméthylcyclohexyle, l'acrylate de t-butylcyclohexyle, l'acrylate de benzyle, l'acrylate d'hydroxyéthyle, l'acrylate d'éthoxyéthyle, l'acrylate d'éthoxyéthoxyéthyle, l'acrylate d'aminoéthyle, l'acrylate de t-butylaminoéthyle, l'acrylate de N,N-diméthylaminoéthyle, l'acrylate de N,N-diéthylaminoéthyle et les méthacrylates correspondants, les amides tels que le diamide d'acide maléique, le diamide d'acide fumarique, l'acrylamide, le N-méthylacrylamide, le N-éthylacrylamide, le N-n-propylacrylamide, le N-isopropylacrylamide, le N-butylacrylamide, le N-octylacrylamide, le N-dodécylacrylamide, le N-octadécylacrylamide, le N-butoxyméthylacrylamide, le N,N-diméthylacrylamide, le N,N-diéthylacrylamide, le N,N-dipropylacrylamide, le N,N-dibutylacrylamide, le N-(N',N'-diméthylamino)éthylacrylamide, le N-(N',N'-diéthylamino)éthylacrylamide, le méthacrylamide et les méthacrylamides N-substitués correspondants, les anhydrides tels que l'anhydride d'acide maléique, les imides tels que l'imide maléique N-acroylbutyro et N-méthacroylbutyro, caprolactame et valérolactame, l'imide maléique N-phényl et N-méthyl, les nitriles tels que par exemple l'acétonitrile et le fumarodinitrile, qui peuvent être présents individuellement ou sous forme de mélanges d'au moins deux de ces composés, et/ou **en ce que** dans le composé de formule I, R², R³ et R⁴ sont identiques et/ou dans le composé de formule I, au moins un des radicaux R², R³ et R⁴ représente l'hydrogène, et/ou **en ce que** les groupes d'acides carboxyliques, les groupes ester d'acides carboxyliques, les groupes anhydride d'acides carboxyliques, les groupes amide d'acides carboxyliques ou les groupes imide d'acides carboxyliques provenant du monomère à insaturation α,β-monoéthylénique sont entièrement ou au moins partiellement hydrolysés sous forme de sels.
